# EUROPEAN PATENT SPECIFICATION

(11) **EP 4 388 990 B1**
(45) Date of publication and mention of the grant of the patent: **12.08.2026**
(21) Application number: 22214540.1
(22) Date of filing: 19.12.2022
(51) Int. Cl.: A61B 5/021, A61B 5/024, A61B 5/11, A61B 5/1171, A61B 5/00

(54) **APPARATUS, METHODS, AND COMPUTER PROGRAMS FOR CONTROLLING USE OF SENSOR DATA**
VORRICHTUNG, VERFAHREN UND COMPUTERPROGRAMME ZUR STEUERUNG DER VERWENDUNG VON SENSORDATEN
APPAREIL, PROCÉDÉS ET PROGRAMMES INFORMATIQUES POUR COMMANDER L'UTILISATION DE DONNÉES DE CAPTEUR

(43) Date of publication of application: 26.06.2024
(73) Proprietor: Omnibuds Ltd, Milton Road Cambridge CB4 0GA (GB)
(72) Inventor: FERLINI, Andrea, Cambridge (GB); MONTANARI, Alessandro, Cambridge (GB); AL-NAIMI, Khaldoon, Beaconsfield (GB)
(74) Representative: Mishcon de Reya LLP

(56) References cited:
- US-A1- 2019 286 233
- US-A1- 2019 320 246

## Description

The present invention relates to apparatus, methods and computer programs for controlling use of sensor data, and in particular, apparatus, methods, and computer programs for controlling use of sensor data in an ear worn device.

Movement of a user's body can have a negative effect on data provided by sensors in devices worn by the user.

It would be desirable to improve use of sensor data provided by sensors in devices worn by a user.

US 2019/32046 A1 and US 2019/286233 A1 disclose biometric monitoring devices, configured to be worn by a subject, including: a physiological sensor configured to detect and/or measure physiological information from the subject, and a processor configured to analyze signals from the physiological sensor to determine if the biometric monitoring device is positioned correctly.

According to an aspect of the present invention there is provided an apparatus comprising means for:
receiving sensor data of a user from a photoplethysmography (PPG) sensor located in an ear-worn device;
determining signal quality of the received sensor data;
comparing the determined signal quality to a quality threshold;
if it is determined that the determined signal quality is greater than the quality threshold, providing the received sensor data to a first processing pipeline for determining, at least, pulse related biometric information; and
if it is determined that the determined signal quality is less than the quality threshold, providing the received sensor data to a second, different processing pipeline for determining, at least, user posture and/or movement related biometric information;
wherein the first processing pipeline is configured to perform at least one of: determining at least one vital sign of the user and user authentication, and wherein the second, different processing pipeline is configured to perform at least one of: facial expression recognition and activity recognition.

In some examples, the means are configured to perform sampling the received sensor data in a temporal window to provide sampled sensor data, wherein determining signal quality is performed on the sampled sensor data.

In some examples, determining signal quality of the received sensor data comprises determining if morphological features of the sensor data are preserved.

In some examples, determining signal quality comprises determining at least one signal metric for the received sensor data.

In some examples, the at least one signal metric comprises at least one of: systolic phase, diastolic phase, ratio between systolic and diastolic phase, pulse width, rise time, perfusion index, dominant frequency, spectral kurtosis, peak-to-peak magnitude variance, and peak-time interval variance.

In some examples, comparing the determined signal quality to a quality threshold comprises comparing the determined at least one signal metric to at least one reference signal metric determined for a user whose face is substantially still and relaxed.

In some examples, the means are configured to:
receive first sensor data from a first PPG sensor located in a first ear-worn device worn on and/or in a first ear of the user;
receive second sensor data from a second PPG sensor located in a second ear-worn device worn on and/or in a second ear of the user; and
determine signal quality of the received first sensor data and the received
second sensor data separately to determine separately to which of the first and second processing pipelines the first sensor data and the second sensor data should be provided.

In some examples, the means comprises
at least one processor; and
at least one memory including computer program code,
the at least one memory storing instructions that, when executed by the at least one processor, cause performance of the apparatus.

According to various, but not necessarily all, embodiments there is provided an electronic device comprising an apparatus as described herein.

According to another aspect of the present invention there is provided a method comprising:
receiving sensor data of a user from a photoplethysmography (PPG) sensor located in an ear-worn device;
determining signal quality of the received sensor data;
comparing the determined signal quality to a quality threshold;
if it is determined that the determined signal quality is greater than the quality threshold, providing the received sensor data to a first processing pipeline for determining, at least, pulse related biometric information; and
if it is determined that the determined signal quality is less than the quality threshold, providing the received sensor data to a second, different processing pipeline for determining, at least, user posture and/or movement related biometric information;
wherein the first processing pipeline performs at least one of: determining at least one vital sign of the user and user authentication, and wherein the second, different processing pipeline performs at least one of: facial expression recognition and activity recognition.

In some examples, the method comprises: sampling the received sensor data in a temporal window to provide sampled sensor data, wherein determining signal quality is performed on the sampled sensor data.

In some examples, determining signal quality of the received sensor data comprises determining if morphological features of the sensor data are preserved.

In some examples, determining signal quality comprises determining at least one signal metric for the received sensor data.

According to another aspect of the present invention there is provided a computer program comprising instructions for causing an apparatus to perform:
receiving sensor data of a user from a photoplethysmography (PPG) sensor located in an ear-worn device;
determining signal quality of the received sensor data;
comparing the determined signal quality to a quality threshold;
if it is determined that the determined signal quality is greater than the quality threshold, providing the received sensor data to a first processing pipeline for determining, at least, pulse related biometric information; and
if it is determined that the determined signal quality is less than the quality threshold, providing the received sensor data to a second, different processing pipeline for determining, at least, user posture and/or movement related biometric information;
wherein the first processing pipeline performs at least one of: determining at least one vital sign of the user and user authentication, and wherein the second, different processing pipeline performs at least one of: facial expression recognition and activity recognition.

In some examples, determining signal quality of the received sensor data comprises determining if morphological features of the sensor data are preserved.

According to various, but not necessarily all, examples there is provided examples as claimed in the appended claims.

According to various, but not necessarily all, embodiments there is provided an apparatus comprising
at least one processor; and
at least one memory including computer program code;
the at least one memory storing instructions that, when executed by the at least one processor, cause the apparatus at least to perform at least a part of one or more methods disclosed herein.

According to various, but not necessarily all, embodiments there is provided an apparatus comprising means for performing at least part of one or more methods disclosed herein.

The description of a function should additionally be considered to also disclose any means suitable for performing that function.

While the above examples of the disclosure and optional features are described separately, it is to be understood that their provision in all possible combinations and permutations is contained within the disclosure. It is to be understood that various examples of the disclosure can comprise any or all of the features described in respect of other examples of the disclosure, and vice versa. Also, it is to be appreciated that any one or more or all of the features, in any combination, may be implemented by/comprised in/performable by an apparatus, a method, and/or computer program instructions as desired, and as appropriate.

Some examples will now be described with reference to the accompanying drawings in which:
FIG. 1 shows an example of the subject matter described herein;
FIG. 2 shows another example of the subject matter described herein;
FIG. 3 shows another example of the subject matter described herein;
FIG. 4 shows another example of the subject matter described herein;
FIG. 5 shows another example of the subject matter described herein;
FIG. 6 shows another example of the subject matter described herein;
FIG. 7 shows another example of the subject matter described herein;
FIG. 8A shows another example of the subject matter described herein; and
FIG 8B shows another example of the subject matter described herein.

The figures are not necessarily to scale. Certain features and views of the figures can be shown schematically or exaggerated in scale in the interest of clarity and conciseness. For example, the dimensions of some elements in the figures can be exaggerated relative to other elements to aid explication. Similar reference numerals are used in the figures to designate similar features. For clarity, all reference numerals are not necessarily displayed in all figures.

Examples of the disclosure relate to apparatus, methods, and/or computer programs for and/or involved in controlling use of sensor data.

The following description and FIGs describe various examples of an apparatus 10 comprising means for:
receiving sensor data 12 of a user from a photoplethysmography (PPG) sensor 14 located in an ear-worn device 16;
determining signal quality of the received sensor data 12;
comparing the determined signal quality to a quality threshold;
if it is determined that the determined signal quality is greater than the quality threshold, providing the received sensor data 12 to a first processing pipeline 18 for determining, at least, pulse related biometric information 20; and if it is determined that the determined signal quality is less than the quality threshold, providing the received sensor data to a second, different processing pipeline 22 for determining, at least, user posture and/or movement related biometric information 26.

The means can comprise at least one processor; and at least one memory including computer program code; the at least one memory storing instructions that, when executed by the at least one processor, cause the performance of the apparatus.

As used herein, an apparatus and/or device and/or component for performing one or more actions should also be considered to disclose an apparatus and/or device and/or component configured to perform the one or more actions.

Similarly, as used herein, an apparatus and/or device and/or component configured to perform one or more actions should also be considered to disclose an apparatus and/or device and/or component for performing the one or more actions.

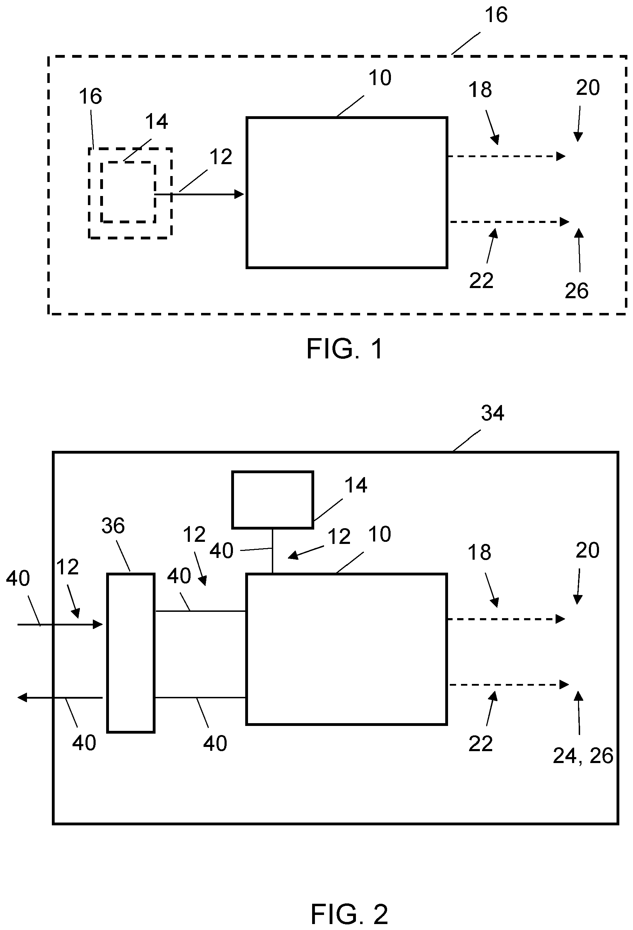

FIG. 1 schematically illustrates an example of an apparatus 10. Various features referred to in relation to FIG. 1 can be found in the other FIGs.

In the example of FIG. 1 the apparatus 10 is configured to receive sensor data 12 of a user from a photoplethysmography (PPG) sensor 14 located in an ear-worn device 16.

The apparatus 10 can be comprised and/or be integrated in a device or devices, which can be considered an electronic device 34 or devices 34. See, for example, FIG. 2.

The apparatus 10 can be comprised in any suitable device or devices.

In some examples, the apparatus 10 is located in an ear-worn device 16, for example the ear-worn device 16 in which the PPG sensor 14 is located.

In some examples, the apparatus 10 is located in a device that is separate from the ear-worn device 16 that comprises the PPG sensor 14. For example, the apparatus 10 can be located in a device, such as a smartphone, to which the ear-worn device 16 is communicatively connected, for example via at least one wired or wireless connection, to allow sensor data 12 from the PPG sensor 14 in the ear-worn device 16 to be received by the apparatus 10.

This is illustrated in the example of FIG. 1 by the dashed boxes indicating the ear-worn device 16.

In the example of FIG. 1, the apparatus 10 is configured to determine signal quality of the received sensor data 12.

Determining signal quality of the received sensor data can comprise determining if morphological features 32 of the sensor data 12 are preserved.

Determining signal quality can comprise determining at least one signal metric for the received sensor data 12.

The at least one signal metric can comprise at least one of: systolic phase, diastolic phase, ratio between systolic and diastolic phase, pulse width, rise time, perfusion index, dominant frequency, spectral kurtosis, peak-to-peak magnitude variance, and peak-time interval variance.

In some examples, the apparatus 10 is configured to perform sampling the received sensor data 12 in a temporal window to provide sampled sensor data 30, wherein determining signal quality is performed on the sampled sensor data 30.

In the example of FIG. 1, the apparatus 10 is configured to compare the determined signal quality to a quality threshold.

Comparing the determined signal quality to a quality threshold can comprise comparing the determined at least one signal metric to at least one reference signal metric determined for a user whose face is substantially still and relaxed.

In the example of FIG. 1, the apparatus 10 is configured to, if it is determined that the determined signal quality is greater than the quality threshold, provide the received sensor data 12 to a first processing pipeline 18 for determining, at least, pulse related biometric information 20, and configured to, if it is determined that the determined signal quality is less than the quality threshold, provide the received sensor data 12 to a second, different processing pipeline 22 for determining, at least, user posture and/or movement related biometric information 26.

The first processing pipeline 18 is configured to perform: at least one of determining at least one vital sign of the user and user authentication, and the second, different processing pipeline is configured to perform at least one of: facial expression recognition and activity recognition.

In examples, the apparatus 10 is configured to perform, and/or cause performance of, and/or control performance of at least part of the first processing pipeline 18 and/or at least part of the second, different processing pipeline 22.

In examples, providing the received sensor data 12 to a first processing pipeline 18 comprises performing, and/or causing performance of, and/or controlling performance of at least part of the first processing pipeline 18.

In examples, providing the received sensor data 12 to a second, different processing pipeline 22 comprises performing, and/or causing performance of, and/or controlling performance of at least part of the second, different processing pipeline 22.

In examples, the apparatus 10 is configured to: receive first sensor data 12 from a first PPG sensor 14 located in a first ear-worn device 16 worn on and/or in a first ear of a user, receive second sensor data 12 from a second PPG sensor 14 located in a second ear-worn device 16 worn on and/or in a second ear of the user, and determine signal quality of the received first sensor data 12 and the received second sensor data 12 separately to determine separately to which of the first and second processing pipelines 18, 22 the first sensor data 12 and the second sensor data 12 should be provided.

FIG. 2 schematically illustrates an example of an electronic device 34.

In the example of FIG. 2, the electronic device 34 comprises an apparatus 10 as described in relation to FIG. 1 and/or as described herein.

Consequently, FIG. 2 illustrates an example of an electronic device 34 comprising an apparatus 10 as described herein.

The electronic device 34 can be or comprise any suitable electronic device 34. For example, the electronic device 34 can be or comprise a control device, and/or an output device and so on.

In examples, the electronic device 34 can be or comprise any suitable personal device, and/or any suitable mobile device, and/or any suitable wearable device and so on. For example, the electronic device 34 can be or comprise an ear-worn device 16 such as at least one headphone, at least one in-ear headphone, at least part of a headset and so on. For example, the electronic device 34 can be or comprise a smartphone.

In examples, the electronic device 34 can be considered an apparatus.

In the example of FIG. 2, the electronic device comprises at least one transceiver 36 and/or at least one PPG sensor 14.

The at least one transceiver 36 can comprise any suitable transceiver 36 or transceivers 36. For example, the at least one transceiver 36 can comprise any suitable transceiver(s) 36 for transmitting and/or receiving one or more signals 40.

In examples, the at least one transceiver 36 is configured to transmit and/or receive one or more signals 40 using wired and/or wireless communication. Any suitable wired and/or wireless communication protocol(s) can be used. For example, W-Fi and/or Bluetooth can be used.

In some examples, one or more separate transmitters and receivers can be used.

In examples, the at least one transceiver 36 is configured to receive one or more signals 40 and transmit to the apparatus 10 the sensor data 12.

For example, the at least one transceiver 36 can receive, from a PPG sensor 14 located in an ear-worn device 16, one or more signals 40 comprising information at least indicative of the sensor data 12 and transmit one or more signals 40 comprising information at least indicative of the sensor data 12 to the apparatus 10.

The at least one PPG sensor 14 can comprise any suitable PPG sensor(s) 14 configured to provide sensor data 12 of a user.

In examples, the electronic device 34 can be or comprise an ear-worn device 16. Accordingly, in examples, the at least one PPG sensor 14 can be at least one PPG sensor 14 located in an ear-worn device 16.

Consequently, in examples, the sensor data 12 can be received by the apparatus 10 from a PPG sensor 14 located in an electronic device 34 with the apparatus 10 and/or can be received by the apparatus 10 from a PPG sensor 14 located separately from an electronic device 34 comprising the apparatus 10.

In examples, the electronic device 34 can comprise any number of additional elements not illustrated in the example of FIG 2. For example, the electronic device 34 can comprise a display.

As illustrated in the example of FIG. 2, the at least one transceiver 36 and the at least one PPG sensor 14 are operationally coupled to the apparatus 10 and any number of intervening elements can exist between them (including no intervening elements).

Additionally, or alternatively, one or more elements of the electronic apparatus 36 illustrated in the example of FIG. 2 can be integrated or combined.

Additionally, or alternatively, one or more elements of the electronic apparatus 36 illustrated in the example of FIG. 2 can be omitted. For example, the at least one transceiver 36 or at least one PPG sensor 14 can be omitted.

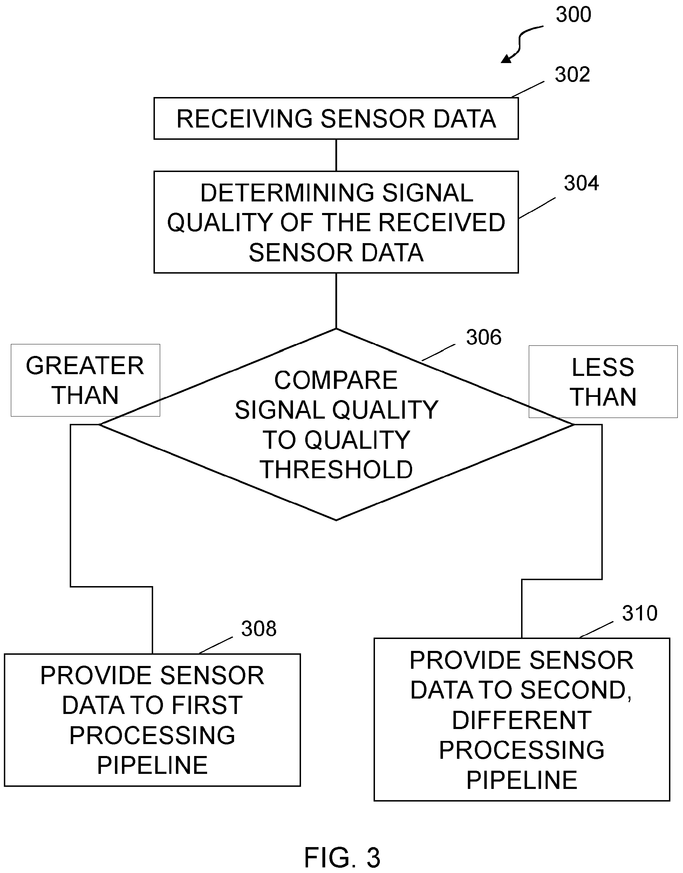

FIG. 3 illustrates an example of a method 300.

One or more of the features discussed in relation to FIG. 3 can be found in one or more of the other FIGs.

In examples, method 300 can be considered a method of controlling use of sensor data.

In examples, method 300 can be considered a method of sensor data control.

In examples, method 300 can be considered a method of improving and/or enhancing use of sensor data.

Method 300 can be performed by any suitable apparatus comprising any suitable means for performing the method 300.

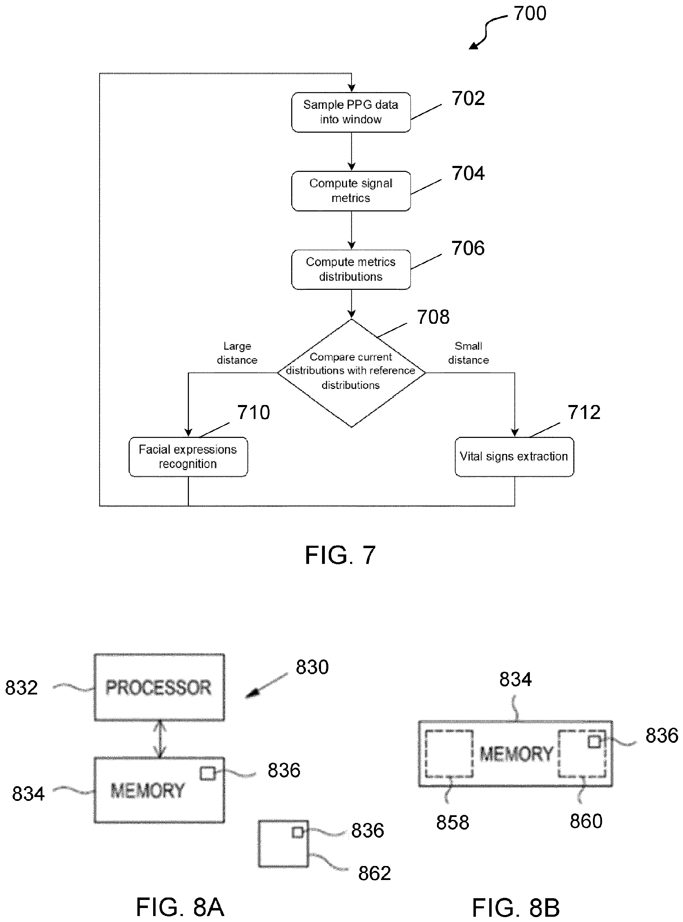

In examples, method 300 can be performed by the apparatus of FIGs 8A and 8B and/or the apparatus of FIG. 1, and/or the electronic device of FIG. 2.

At block 302, method 300 comprises receiving sensor data 12 of a user from a photoplethysmography (PPG) sensor 14 located in an ear-worn device 16.

Block 302 can be performed in any suitable way, using any suitable method. For example, block 302 can comprise receiving sensor data 12 of a user, from any suitable PPG sensor 14, in any suitable way, located in any suitable ear-worn device 16. See, for example, FIG. 1 and/or FIG. 2.

Sensor data 12 can comprise any suitable data/information produced, and/or generated by a PPG sensor 14. In examples, sensor data 12 can be considered PPG data.

In examples, a user can be considered a person who is wearing the ear-worn device 16 in which the PPG sensor 14 is located.

Sensor data 12 of a user can be considered data/information determined using a PPG sensor 14 for a user. For example, sensor data 12 can be considered to be sensor data 21 of a user because the sensor data 12 has been determined in relation to the user using the PPG sensor 14.

In examples, sensor data 12 of a user can be considered sensor data 12 for a user, and/or sensor data 12 determined from a user, and/or sensor data 12 relating to a user and so on.

An ear-worn device 16 can be considered a device worn on and/or in at least one ear of the user.

For example, an ear-worn device 16 can comprise an over-ear headphone, an on-ear headphone, an in-ear headphone (commonly referred to as an earbud) and so on.

In examples, different parts of a pair of headphones can be considered different ear-worn devices 16. For example, a first ear cup of a pair of headphones can be considered a first ear-worn device 16 and a second ear cup of a pair of headphones can be considered a second ear-worn device 16.

For example, a first in-ear headphone of a set of in-ear headphones can be considered a first ear-worn device 16 and a second in-ear headphone of a set of in-ear headphones can be considered a second ear-worn device 16.

A PPG sensor 14 can be considered to be located in an ear-worn device 16 because it is comprised and/or integrated in the ear-worn device 16 and/or forms part of the ear-worn device 16.

At block 304, method 300 comprises determining signal quality of the received sensor data 12.

Block 304 can be performed in any suitable way using any suitable method. For example, any suitable method to determine signal quality of the received sensor data 12 can be used.

In examples, signal quality can be considered a measure of how affected the sensor data is by artifacts, such as motion induced artifacts, and/or corruption, and/or noise and so on. For example, the inventors have realized that facial expressions made by a user can affect quality of PPG sensor data. For example, the inventors have realized that facial expressions of a user can affect morphological features of PPG sensor data.

Accordingly, in examples, determining signal quality of the received sensor data 12 can be considered determining how affected the sensor data is by artifacts, such as motion induced artifacts, and/or corruption, and/or noise and so on.

For example, the inventors have realized that sensor data 12 of a user from a PPG sensor 14 located in an ear-worn device 16 can be significantly affected by facial expressions made by the user. That is, the inventors have realized that signal quality of sensor data from a PPG sensor 14 located in an ear-worn device 16 can be deteriorated by the user making facial expressions.

In some examples, method 300 comprises sampling the received sensor data 12 in a temporal window 28 to provide sampled sensor data 30, wherein determining signal quality is performed on the sampled sensor data 30.

A temporal window 28 of any suitable length can be used. For example, the temporal window 28 can have a length in the range 10 seconds to 20 seconds.

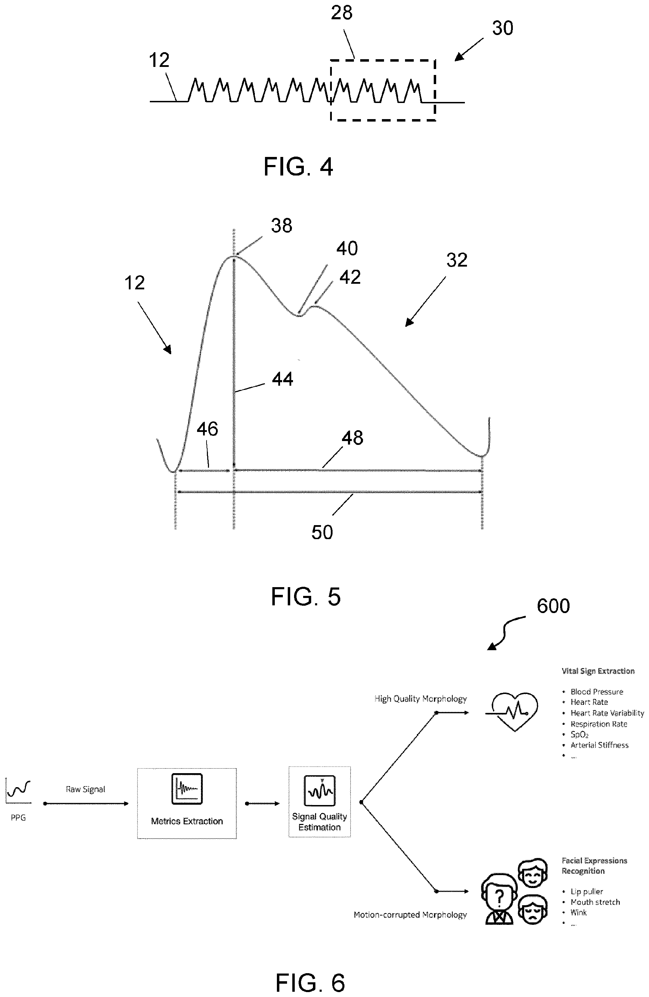

By way of example, reference is made to the example of FIG. 4.

FIG. 4 schematically illustrates an example of sensor data 12. In the example of FIG. 4, a temporal window 28 is shown encompassing a portion of the sensor data 28.

In the illustrated example, the portion of the sensor data 28 in the temporal window 28 can be considered sampled sensor data 30 and determining signal quality is performed on the sampled sensor data 30.

In examples, different portions of the sensor data 12 will be analysed in the temporal window 28. For example, in the example of FIG. 4, the temporal window 28 can be moved to the left to encompass a different portion of the sensor data 12 to allow signal quality of a different portion of the sensor data 12 to be determined.

Returning to the example of FIG. 3, determining signal quality of the received sensor data 12 can comprise determining if the received sensor data 12 has a form expected for PPG sensor data.

In some examples, determining signal quality of the received sensor data 12 comprises determining if morphological features 32 of the sensor data 12 are preserved.

Determining if morphological features 32 of the sensor data 12 are preserved can be performed in any suitable way using any suitable method.

In examples, determining if morphological features 32 of the sensor data 12 are preserved can be considered determining if one or more morphological features 32 of the sensor data 12 have been affected, and/or corrupted, and/or changed, and/or altered and so on.

In some examples, determining if morphological features 32 of the sensor data 12 are preserved comprises determining how much one or more morphological features 32 of the sensor data 12 are affected by artifacts, such as motion induced artifacts, and/or corruption, and/or noise and so on.

Morphological features 32 of the sensor data 12 can be considered any suitable feature or features relating to the form, and/or shape, and/or structure of the signal represented by the sensor data 12. By way of example, reference is made to the example of FIG. 5.

FIG. 5 illustrates a visual representation of morphological features 32 of sensor data 12 from a PPG sensor 14. Fig. 5 can be considered to illustrate a visual representation of morphological features 32 of a PPG signal.

FIG. 5 can be considered to illustrate a single pulse wave of a PPG signal. In the example of FIG. 5 various morphological features are illustrated. In the illustrated example, systolic peak 38, dicrotic notch 40, diastolic peak 42, pulse wave amplitude 44, rise time 46, fall time 48 and pulse width 50 are indicated.

Returning to the example of FIG. 3, in some examples determining signal quality comprises determining at least one signal metric for the received sensor data 12.

Determining at least one signal metric can be performed in any suitable way using any suitable method.

Any suitable signal metric or metrics can be determined. For example, any suitable signal metric or metrics for use in determining signal quality of the sensor data 12 can be used. For example, any suitable signal metric or metrics for use in assessing the morphology of the PPG wave in the sensor data 12 can be used.

The at least one signal metric can comprise at least one of:
Systolic phase: The Amplitude of the systolic peak and the time at which the systolic peak is located in the PPG signal,
Diastolic phase: The Amplitude of the diastolic peak and the time at which the diastolic peak is located in the PPG signal,
Ratio between systolic and diastolic phase: It is an indicator of abnormalities in blood pressure. It is also referred to as Augmentation index or Reflection index,
Pulse width: It is the time between the beginning and end of a PPG pulse wave. It correlates with our heart's systemic vascular resistance,
Rise time: The time between the foot of the PPG pulse and the systolic peak, Perfusion index (P1): Plis the ratio of the pulsatile blood flow (AC component) to the non-pulsatile or static blood in peripheral tissue (DC component),
Dominant frequency: The dominant frequency of PPG signal can be useful to give insights into the presence of artifacts at a different frequency outside the heart rate frequency band [0.4,4Hz],
Spectral Kurtosis: Also known as Frequency Domain Kurtosis, describes the distribution of the observed PPG signal frequencies around the mean,
Peak-to-peak magnitude variance: It is the variance of the difference between the pulse wave amplitude between two adjacent pulse waves,
Peak-Time interval variance: It is the variance of the pulse width between peaks of two adjacent PPG waves.

Accordingly, in examples, the at least one signal metric comprises at least one of:
systolic phase, diastolic phase, ratio between systolic and diastolic phase, pulse width, rise time, perfusion index, dominant frequency, spectral kurtosis, peak-to-peak magnitude variance, and peak-time interval variance.

Determining signal quality can comprise determining at least one signal metric for the sampled sensor data 30 from the temporal window 28. In some examples, this can allow a distribution for at least one signal metric to be determined for the sampled sensor data. In examples, a distribution for at least one signal metric can be considered at least one signal metric.

At block 306, method 300 comprises comparing the determined signal quality to a quality threshold.

Block 306 can be performed in any suitable way using any suitable method. For example, comparing the determined signal quality to a quality threshold can be performed in any suitable way to allow a determination to be made as to what use the sensor data 12 is suitable for.

Any suitable quality threshold having any suitable value can be used. In examples, the quality threshold can be determined via experimentation to allow a suitable threshold for different uses of the sensor data 12 to be determined. See, for example, blocks 308 and 310.

Accordingly, in examples, the quality threshold can be determined based, at least in part, on the potential uses of the sensor data 12. For example, the quality threshold can be determined based, at least in part, on the different processing pipelines to which the sensor data 12 can be provided to ensure that the quality of the sensor data is sufficient for the processing pipeline chosen to which to provide the sensor data 12.

In examples, comparing the determined signal quality to a quality threshold can comprise determining if the determined signal quality of the sensor data 12 can be considered high or low. In examples, the threshold can be determined at a suitable point to divide the quality of the sensor data 12 appropriately with regard to the potential uses of the sensor data 12.

In some examples, comparing the determined signal quality to a quality threshold comprises comparing at least one metric and/or at least one metric distribution to at least one reference metric and/or at least one reference metric distribution.

In examples, the at least one reference metric and/or at least one reference metric distribution can be metric(s) and/or metric distribution(s) determined for PPG sensor data 12 having preserved and/or undisturbed morphological features 32.

In some examples, comparing the determined signal quality to a quality threshold comprises comparing the determined at least one signal metric to at least one reference signal metric determined for a user whose face is substantially still and relaxed.

The at least one reference signal metric can be determined in any suitable way, using any suitable method. For example, the at least one reference signal metric can be predetermined and stored on the electronic device 34. For example, the at least one reference signal metric can be determined by performing a calibration routine with a user while the user's face is substantially still and relaxed.

In some examples, comparing the determined signal quality to a quality threshold comprises determining the distance between at least one signal metric data distribution, for example for sampled sensor data 30, and a reference signal metric distribution (for example, in the "still" condition for a user). In examples, a reference signal metric distribution can be considered at least one reference signal metric.

Any suitable method to determine the distance between two distributions can be used. For example, the Kolmogorov-Smirnov test can be used, which returns the KS statistic giving an indication of how distant the two distributions are. Other applicable methods include, but are not limited to, Kullback-Leib er divergence and Wasserstein distance.

In examples, a smaller distance between the at least one signal metric data distribution and a reference data distribution for a user whose face is substantially still and relaxed indicates higher signal quality and a larger distance between the at least one signal metric and a reference data distribution for a user whose face is substantially still and relaxed indicates lower signal quality.

Any suitable distance threshold between the distributions can be used.

In examples, the distance threshold is based, at least in part, on the metric(s) extracted from the still reference data distribution.

For example, metric "Ratio between systolic and diastolic phase" can be extracted from still reference data distribution, the same metric can be extracted from the sensor data, which can be considered data that is being collected *"live".* The distance between these two can be determined and compared against an application dependent threshold.

For example, applications like blood pressure calculation require the user to be very still in order to be accurate, hence the threshold would be stricter in such situation.

This can be done for the other metrics listed herein.

In some examples, comparing the determined signal quality to a quality threshold comprises determining a weighted sum of all distances between the at least one metric distribution and corresponding reference data distribution(s) and then applying a threshold on it.

For example, w1 * dl + w2 * d2 + ... where dx is the distance between the reference distribution and the metric x distribution and wx is the corresponding weight.

The corresponding weights can be determined in any suitable way using any suitable method. In some examples, the corresponding weights can be determined by experiment. For example, greater weight can be applied to the metric or metrics determined to be more strongly indicative of signal quality.

In some examples, block 304 and 306 can be combined and/or block 306 can be considered to form part of block 304.

For example, in some examples, determining signal quality can be considered to comprise comparing the determined at least one signal metric to at least one reference signal metric determined for a user whose face is substantially still and relaxed.

If it is determined, at block 306, that the determined signal quality is greater than the quality threshold, method 300 proceeds to block 308 and if it is determined, at block 306, that the determined signal quality is less than the quality threshold, method 300 proceeds to block 310.

In examples where comparing the determined signal quality to a quality threshold comprises determining the distance between at least one signal metric data distribution, for example for sampled sensor data 30, and a reference signal metric distribution, determining if the signal quality is greater than a quality threshold can
comprise determining if the distance between the distributions is less than a value.

Similarly, in such examples, determining if the signal quality is less than a quality threshold can comprise determining if the distance between the distributions is greater than a value.

At block 308, method 300 comprises providing the received sensor data 12 to a first processing pipeline 18 for determining, at least, pulse related biometric information 20.

In examples, at block 308, method 300 comprises providing the received sensor data 12 to the first processing pipeline 18 and not the second, different processing pipeline 22.

At block 310, method 300 comprises providing the received sensor data 12 to a second, different processing pipeline 22 for determining, at least, user posture and/or movement related biometric information 26.

In examples, at block 310, method 300 comprises providing the received sensor data 12 to the second, different processing pipeline 22 and not the first processing pipeline 18.

Consequently, FIG. 3 illustrates a method 300 comprising:
receiving sensor data 12 of a user from a photoplethysmography (PPG) sensor 14 located in an ear-worn device 16;
determining signal quality of the received sensor data 12;
comparing the determined signal quality to a quality threshold;
if it is determined that the determined signal quality is greater than the quality threshold, providing the received sensor data 12 to a first processing pipeline 18 for determining, at least, pulse related biometric information 20; and
if it is determined that the determined signal quality is less than the quality threshold, providing the received sensor data to a second, different processing pipeline 22 for determining, at least, user posture and/or movement related biometric information 26.

Providing the received sensor data 12 to a processing pipeline 18, 22 can be performed in any suitable way, using any suitable method.

In examples, providing the received sensor data 12 to a processing pipeline 18, 22 comprises transmitting the received sensor data 12.

In examples, providing the received sensor data 23 to a processing pipeline 18, 22 comprises performing, and/or causing performance of, and/or controlling performance of at least part of the processing pipeline 18, 22.

Accordingly, in some examples, providing the received sensor data 12 to a first processing pipeline 18 comprises performing, and/or causing performance of, and/or controlling performance of at least part of the first processing pipeline 18.

Accordingly, in examples, providing the received sensor data 12 to a second, different processing pipeline 22 comprises performing, and/or causing performance of, and/or controlling performance of at least part of the second, different processing pipeline 22.

In examples, a processing pipeline 18, 22 can be considered a processing channel, and/or a processing sequence and so on.

A processing pipeline 18, 22 can be considered to comprise at least one processing operation, and/or processing action, and/or processing procedure.

In examples, the pulse related biometric information 20 can be considered any biometric information of a user determined based, at least in part, on pulse information in the received sensor data 12 and/or related to a pulse of the user.

In examples, the first processing pipeline 18 comprises a processing pipeline that requires high or higher quality sensor data to produce accurate results, for example sensor data 12 having substantially preserved and/or undisturbed morphological features 32 and/or good signal to noise ratio and so on.

Accordingly, the sensor data 12 can be provided to the first processing pipeline 18 based, at least in part, on amount of degradation of morphological features 32, and/or signal to noise ratio and so on.

Accordingly, in examples, the pulse related biometric information 20 can be considered biometric information of a user determined based, at least in part, on pulse information in the received sensor data 12 having substantially preserved and/or undisturbed morphological features 32.

In examples, the pulse related biometric information 20 comprises at least one vital sign of the user. For example, the pulse related biometric information 20 can comprise at least one of: blood pressure, heart rate, heart rate variability, respiration rate, Sp02, arterial stiffness and so on.

Accordingly, in examples, the first processing pipeline 18 is configured to determine at least one vital sign of the user.

In examples, the user posture and/or movement related biometric information 26 can be considered any biometric information of the user associated with and/or related the posture and/or movement of at least part of a user's body.

In examples, the second, different processing pipeline 22 comprises a processing pipeline that does not require high or higher quality sensor data to operate, for example the second, different processing pipeline 22 does not require sensor data 12 having substantially preserved and/or undisturbed morphological features 32.

In examples, the user posture and/or movement related biometric information 26 comprises information of movement of the user's head and/or face. In examples, the user posture and/or movement related biometric information 26 comprises information of a facial expression made by the user.

Accordingly, in examples, the second, different processing pipeline 22 is configured to recognize at least one facial expression of a user.

The first processing pipeline 18 is configured to perform at least one of: determining at least one vital sign of the user and user authentication, and the second, different processing pipeline 22 is configured to perform at least one of: facial recognition and activity recognition.

In examples, method 300 can be repeated for sensor data 12 being provided by a PPG sensor 14 located in an ear-worn device 16. For example, method 300 can proceed from block 308 or 310 back to block 302.

In some examples, at least part of method 300 can be performed separately for sensor data 12 from different PPG sensors 14 located in ear-worn devices 16 worn on different ears of the user.

In examples, method 300 comprises receiving first sensor data 12 from a first PPG sensor 14 located in a first ear-worn device 16 worn on and/or in a first ear of the user, receiving second sensor data 12 from a second PPG sensor 14 located in a second ear-worn device 16 worn on and/or in a second ear of the user, and determining signal quality of the received first sensor data 12 and the received second sensor data 12 separately to determine to which of the first and second processing pipelines 18, 22 the first sensor data 12 and the second sensor data 12 should be provided.

This is advantageous as, for example, it allows good data from a first or second PPG sensor 14 to be used for, for example, vital sign determination if possible.

In some examples, method 300 can comprise comparing the signal quality to a quality threshold and routing the sensor data 12 to the first or second processing pipeline 18, 22 based, at least in part, on the comparison.

Examples of the disclosure are advantageous and/or provide technical benefits.

For example, examples of the disclosure allow PPG sensor data to be provided to and used by an appropriate processing pipeline and avoids, for example, discarding sensor data having lower signal quality.

For example, examples of the disclosure allow PPG sensor data to be used for vital sign determination when the signal quality is high indicating that the user is not making a strong facial expression and allows PPG sensor data to be used for facial expression recognition when the signal quality is low indicating that the user is making a strong facial expression. This allows, for example, high accuracy vital sign information to be determined and also provides for performing facial expression recognition when it is likely that there is a strong facial expression to be recognized.

For example, examples of the disclosure allow sensor data from different PPG sensors in ear-worn devices worn by the user to be treated differently, which, for example, allows for sensor data from the different PPG sensors to be treated appropriately when, for example, a facial expression of a user affects the sensor data from one PPG sensor more than the sensor data from a second PPG sensor.

FIG. 6 illustrates an example overview of examples of the disclosure. FIG. 6 can be considered to illustrate an example of a method 600.

Method 600 can be performed by any suitable apparatus comprising any suitable means for performing the method 600.

In examples, method 600 can be performed by the apparatus of FIGs 8A and 8B and/or the apparatus of FIG. 1, and/or the electronic device of FIG. 2.

Based on the observations made by the inventors that, for example, facial expressions of a user can affect sensor data 12 from a PPG sensor 14 located in an
ear-worn device 16, FIG. 6 illustrates an overview of examples of the disclosure that tries to make the best use of the PPG signal (both corrupted and not corrupted) by ensuring high accuracy vital signs measurement and enabling the detection of facial expressions.

Examples of the disclosure can detect at the individual scale whether the PPG data
is affected by facial-expressions-induced motion artifacts. To do so, some examples of the disclosure leverage a set of features of the PPG signal (computed in real-time on the ear-worn wearable device that is collecting the PPG data) to determine whether each PPG wave (valley-peak-valley) is suitable for extracting reliable vitals or not.

This way, examples of the disclosure first make sure the vital signs extraction is carried out only under favourable circumstances, preventing the inevitable lower quality/unreliable estimates due to motion artifacts.

At the same time, to make sure examples of the disclosure do not waste the precious resources used to collect PPG data, rather than discarding facial-expressions-corrupted PPG signal, examples of the disclosure leverage the same metrics used to assess the quality of the PPG wave morphology to recognize the facial expression the user is performing.

In examples, this allows recognising even those subtle facial expressions that show very low magnitude on IMUs). Ultimately, this can enable, for example, several context/behavioural/mood- aware applications.

FIG. 7 illustrates an example of a method 700.

Method 700 can be performed by any suitable apparatus comprising any suitable means for performing the method 700.

In examples, method 700 can be performed by the apparatus of FIGs 8A and 8B and/or the apparatus of FIG. 1, and/or the electronic device of FIG. 2.

Block 702 involves sampling the PPG sensor and storing the data in a small window (temporary buffer) of a few seconds (e.g., 10 to 20 seconds). Then signal metrics are computed on this window (block 704). The list indicated above in relation to FIG. 3 describes in more detail some of the PPG metrics that can be used to assess the morphology of the PPG wave. Additional metrics can be also added.

In examples, these are the same metrics that can be used to classify the various head and facial expressions. In examples, they are simple signal processing metrics that can be computed directly on in-ear headphones with limited overhead in terms of computation and energy usage (see Metrics Extraction block in FIG. 6). A visual representation of these features can be found in the example of FIG. 5.

In examples, method 700 comprises determining whether the morphological features of the PPG wave are preserved (or altered in a minimal way which would not impact the quality and reliability of the vital signs). In examples, this relies on the fact that the features presented above will have very different values when the PPG signal is clean and when it is corrupted. To achieve this the method first computes statistical distributions for each metric (block 706).

Examples of the disclosure leverages this difference to determine if the current PPG signal is clean, i.e., similar to the "still" condition, or if it is affected by motion artifacts. In examples, this is achieved by measuring the distance between the current data distribution and the reference data distribution (i.e. in the "still" condition). There are various ways to determine the distance between two distributions. A simple but effective way is to use the Kolmogorov-Smirnov test which returns the KS statistic giving an indication of how distant the two distributions are. Other applicable methods include, but are not limited to, Kullback-Leib er divergence and Wassersteindistance.

Once the distance between the reference distribution and the current distribution is determined for all metrics, in the example of FIG. 7, a decision on how to use the PPG data is made (block 708).

If the distance is small enough it means that the current PPG signal is clean and can be used to estimate vital signs reliably because the morphological features 32 of the signal are preserved.

If the distance is large instead, it means that the morphological features 32 are corrupted, and the signal cannot be used for vital signs estimation. In this case, the signal is used to recognise what facial expression or motion was performed avoiding dropping the signal completely.

One option that can be used to take this decision is to compute the weighted sum of all distances and then apply a threshold on it. For example, w1 * dl + w2 * d2 + ...
where dx is the distance between the reference distribution and the metric x and wx is the corresponding weight.

Other approaches could also be applied, for example feeding the set of distance values to machine learning models like support vector machines, random forests or neural networks.

In examples, the method 700 stores the distribution of clean PPG signal, to use it as a reference to compare against the current distribution. This reference distribution can be programmed into the component offline (i.e., at the factory) or it can be determined the first time the user uses the device, asking him/her to stay still for a few seconds. The storage required to store the reference distribution is very limited (in order of tens of bytes) hence suitable to be stored directly on constrained devices like ear-worn wearable devices.

In examples, method 700 could run on either of multiple ear-worn devices 16 (left and right) if they are both equipped with a PPG sensor 14 or on only a single device if the PPG sensor 14 is available on one side only or if the power consumption between left and right in-ear headphones needs to be balanced.

Since the effect of facial expression on PPG signals might not be symmetric between left and right, examples of the disclosure can benefit from running on both in-ear headphones by for example extracting vitals from the uncorrupted signal of one ear whilst recognizing facial expressions from the corrupted signal recorded from the other ear.

In examples, if the quality of the current signal is deemed to be good, examples of the disclosure extract high-quality, reliable, vital signs from the PPG trace by leveraging existing, well-established, signal processing pipelines.

To perform facial expression recognition, examples of the disclosure support both machine learning and deep learning models.

In other examples additional branches after the signal quality estimation (FIG. 6) can be added to perform other applications. For example, high quality PPG morphology can be used for user authentication, whilst from low quality PPG morphology it would be possible to perform activity recognition.

Fig 8A illustrates an example of a controller 830 suitable for use in an apparatus, such as apparatus 10 of FIG. 1 and/or FIG. 2. In examples, controller 830 can be considered an apparatus 10.

Implementation of a controller 830 may be as controller circuitry. The controller 830 may be implemented in hardware alone, have certain aspects in software including firmware alone or can be a combination of hardware and software (including firmware).

As illustrated in Fig 8A the controller 830 may be implemented using instructions that enable hardware functionality, for example, by using executable instructions of a computer program 836 in a general-purpose or special-purpose processor 832 that may be stored on a computer readable storage medium (disk, memory etc.) to be executed by such a processor 832.

The processor 832 is configured to read from and write to the memory 834. The processor 832 may also comprise an output interface via which data and/or commands are output by the processor 832 and an input interface via which data and/or commands are input to the processor 832.

The memory 834 stores a computer program 836 comprising computer program instructions (computer program code) that controls the operation of the apparatus when loaded into the processor 832. The computer program instructions, of the computer program 836, provide the logic and routines that enables the apparatus to perform the methods illustrated in the accompanying Figs. The processor 832 by reading the memory 834 is able to load and execute the computer program 836.

The apparatus comprises:
at least one processor 832; and
at least one memory 834 including computer program code
the at least one memory 834 and the computer program code configured to, with the at least one processor 832, cause the apparatus at least to perform:
   receiving sensor data 12 of a user from a photoplethysmography (PPG) sensor 14 located in an ear-worn device 16;
   determining signal quality of the received sensor data 12;
   comparing the determined signal quality to a quality threshold;
   if it is determined that the determined signal quality is greater than the quality threshold, providing the received sensor data 12 to a first processing pipeline 18 for determining, at least, pulse related biometric information 20; and
   if it is determined that the determined signal quality is less than the quality threshold, providing the received sensor data to a second, different processing pipeline 22 for determining, at least, user posture and/or movement related biometric information 26.

The apparatus comprises:
at least one processor 832; and
at least one memory 834 including computer program code,
the at least one memory storing instructions that, when executed by the at least one processor 832, cause the apparatus at least to:
   receiving sensor data 12 of a user from a photoplethysmography (PPG) sensor 14 located in an ear-worn device 16;
   determining signal quality of the received sensor data 12;
   comparing the determined signal quality to a quality threshold;
   if it is determined that the determined signal quality is greater than the quality threshold, providing the received sensor data 12 to a first processing pipeline 18 for determining, at least, pulse related biometric information 20; and
   if it is determined that the determined signal quality is less than the quality threshold, providing the received sensor data to a second, different processing pipeline 22 for determining, at least, user posture and/or movement related biometric information 26.

As illustrated in Fig 8A, the computer program 836 may arrive at the apparatus via any suitable delivery mechanism 862. The delivery mechanism 862 may be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid-state memory, an article of manufacture that comprises or tangibly embodies the computer program 836. The delivery mechanism may be a signal configured to reliably transfer the computer program 836. The apparatus may propagate or transmit the computer program 836 as a computer data signal.

Computer program instructions for causing an apparatus to perform at least the following or for performing at least the following:
receiving sensor data 12 of a user from a photoplethysmography (PPG) sensor 14 located in an ear-worn device 16;
determining signal quality of the received sensor data 12;
comparing the determined signal quality to a quality threshold;
if it is determined that the determined signal quality is greater than the quality threshold, providing the received sensor data 12 to a first processing pipeline 18 for determining, at least, pulse related biometric information 20; and
if it is determined that the determined signal quality is less than the quality threshold, providing the received sensor data to a second, different processing pipeline 22 for determining, at least, user posture and/or movement related biometric information 26.

The computer program instructions may be comprised in a computer program, a non-transitory computer readable medium, a computer program product, a machine readable medium. In some but not necessarily all examples, the computer program instructions may be distributed over more than one computer program.

Although the memory 834 is illustrated as a single component/circuitry it may be implemented as one or more separate components/circuitry some or all of which may be integrated/removable and/or may provide permanent/semi-permanent/ dynamic/cached storage.

In examples the memory 834 comprises a random-access memory 858 and a read only memory 860. In examples the computer program 836 can be stored in the read only memory 858. See, for example, Fig. 8B.

In examples the memory 834 can be split into random access memory 858 and read only memory 860.

Although the processor 832 is illustrated as a single component/circuitry it may be implemented as one or more separate components/circuitry some or all of which may be integrated/removable. The processor 832 may be a single core or multi-core processor.

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixedfunction device, gate array or programmable logic device etc.

As used in this application, the term 'circuitry' may refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory or memories that work together to cause an apparatus, such as a mobile phone or server, to perform various functions and
(c) hardware circuit(s) and or processor(s), such as a microprocessor(s) or a portion of a microprocessor(s), that requires software (for example, firmware) for operation,
but the software may not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

The blocks illustrated in the accompanying Figs may represent steps in a method and/or sections of code in the computer program 836. The illustration of a particular order to the blocks does not necessarily imply that there is a required or preferred order for the blocks and the order and arrangement of the block may be varied. Furthermore, it may be possible for some blocks to be omitted.

Where a structural feature has been described, it may be replaced by means for performing one or more of the functions of the structural feature whether that function or those functions are explicitly or implicitly described.

Thus, the apparatus can comprise means for:
receiving sensor data 12 of a user from a photoplethysmography (PPG) sensor 14 located in an ear-worn device 16;
determining signal quality of the received sensor data 12;
comparing the determined signal quality to a quality threshold;
if it is determined that the determined signal quality is greater than the quality threshold, providing the received sensor data 12 to a first processing pipeline 18 for determining, at least, pulse related biometric information 20; and
if it is determined that the determined signal quality is less than the quality threshold, providing the received sensor data to a second, different processing pipeline 22 for determining, at least, user posture and/or movement related biometric information 26.

In examples, an apparatus can comprise means for performing one or more methods, and/or at least part of one or more methods, as disclosed herein.

In examples, an apparatus can be configured to perform one or more methods, and/or at least part of one or more methods, as disclosed herein.

The apparatus can be provided in an electronic device, for example, a mobile terminal, according to an example of the present disclosure. It should be understood, however, that a mobile terminal is merely illustrative of an electronic device that would benefit from examples of implementations of the present disclosure and, therefore, should not be taken to limit the scope of the present disclosure to the same.

While in certain implementation examples, the apparatus can be provided in a mobile terminal, other types of electronic devices, such as, but not limited to: mobile communication devices, hand portable electronic devices, wearable computing devices, portable digital assistants (PDAs), pagers, mobile computers, desktop computers, televisions, gaming devices, laptop computers, cameras, video recorders, GPS devices and other types of electronic systems, can readily employ examples of the present disclosure. Furthermore, devices can readily employ examples of the present disclosure regardless of their intent to provide mobility.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one..." or by using "consisting".

In this description, the wording 'connect', 'couple' and 'communication' and their derivatives mean operationally connected/coupled/in communication. It should be appreciated that any number or combination of intervening components can exist (including no intervening components), i.e., so as to provide direct or indirect connection/coupling/communication. Any such intervening components can include hardware and/or software components.

As used herein, the term "determine/determining" (and grammatical variants thereof) can include, not least: calculating, computing, processing, deriving, measuring, investigating, identifying, looking up (for example, looking up in a table, a database or another data structure), ascertaining and the like. Also, "determining" can include receiving (for example, receiving information), accessing (for example, accessing data in a memory), obtaining and the like. Also, " determine/determining" can include resolving, selecting, choosing, establishing, and the like.

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples.

Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although examples have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not. Although features have been described with reference to certain examples, those features may also be present in other examples whether described or not.

The term 'a', 'an' or 'the' is used in this document with an inclusive not an exclusive meaning.

That is any reference to X comprising a/an/the Y indicates that X may comprise only one Y or may comprise more than one Y unless the context clearly indicates the contrary. If it is intended to use 'a', 'an' or 'the' with an exclusive meaning then it will be made clear in the context. In some circumstances the use of 'at least one' or 'one or more' may be used to emphasis an inclusive meaning but the absence of these terms should not be taken to infer any exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

## Claims

1. An apparatus (10) comprising means for:
receiving sensor data (12) of a user from a photoplethysmography, PPG, sensor (14) located in an ear-worn device (16);
determining signal quality of the received sensor data (12);
comparing the determined signal quality to a quality threshold;
if it is determined that the determined signal quality is greater than the quality threshold, providing the received sensor data (12) to a first processing pipeline (18) for determining, at least, pulse related biometric information (20); and
if it is determined that the determined signal quality is less than the quality threshold, providing the received sensor data to a second, different processing pipeline (22) for determining, at least, user posture and/or movement related biometric information (26);
wherein the first processing pipeline is configured to perform at least one of:
determining at least one vital sign of the user and user authentication, and wherein the second, different processing pipeline is configured to perform at least one of: facial expression recognition and activity recognition.

2. An apparatus as claimed in claim 1, wherein the means are configured to perform sampling the received sensor data (12) in a temporal window to provide sampled sensor data (30), wherein determining signal quality is performed on the sampled sensor data.

3. An apparatus as claimed in any preceding claim, wherein determining signal quality of the received sensor data comprises determining if morphological features (32) of the sensor data (12) are preserved.

4. An apparatus as claimed in any preceding claim, wherein determining signal quality comprises determining at least one signal metric for the received sensor data (12).

5. An apparatus as claimed in any preceding claim, wherein the at least one signal metric comprises at least one of: systolic phase, diastolic phase, ratio between systolic and diastolic phase, pulse width, rise time, perfusion index, dominant frequency, spectral kurtosis, peak-to-peak magnitude variance, and peak-time interval variance.

6. An apparatus as claimed in claim 4 or 5, wherein comparing the determined signal quality to a quality threshold comprises comparing the determined at least one signal metric to at least one reference signal metric determined for a user whose face is substantially still and relaxed.

7. An apparatus as claimed in any preceding claim, wherein the means are configured to:
receive first sensor data (12) from a first PPG sensor (14) located in a first ear-worn device (16) worn on and/or in a first ear of the user;
receive second sensor data (12) from a second PPG sensor (14) located in a second ear-worn device (16) worn on and/or in a second ear of the user; and
determine signal quality of the received first sensor data and the received second sensor data separately to determine separately to which of the first and second processing pipelines (18, 22) the first sensor data and the second sensor data should be provided.

8. An electronic device (34) comprising an apparatus (10) as claimed in at least one of claims 1 to 7.

9. A method (300) comprising:
receiving (302) sensor data (12) of a user from a photoplethysmography, PPG, sensor (14) located in an ear-worn device (16);
determining (304) signal quality of the received sensor data (12);
comparing (306) the determined signal quality to a quality threshold;
if it is determined that the determined signal quality is greater than the quality threshold, providing (308) the received sensor data (12) to a first processing pipeline (18) for determining, at least, pulse related biometric information (20); and
if it is determined that the determined signal quality is less than the quality threshold, providing (310) the received sensor data (12) to a second, different processing pipeline (22) for determining, at least, user posture and/or movement related biometric information (26);
wherein the first processing pipeline performs at least one of: determining at least one vital sign of the user and user authentication, and wherein the second, different processing pipeline performs at least one of: facial expression recognition and activity recognition.

10. A method as claimed in claim 9, comprising:
sampling the received sensor data (12) in a temporal window to provide sampled sensor data (30), wherein determining signal quality is performed on the sampled sensor data.

11. A method as claimed in claim 9 or 10, wherein determining signal quality of the received sensor data comprises determining if morphological features (32) of the sensor data (12) are preserved.

12. A method as claimed in claim 9, 10 or 11, wherein determining signal quality comprises determining at least one signal metric for the received sensor data (12).

13. A computer program comprising instructions for causing an apparatus (10) to perform:
receiving sensor data (12) of a user from a photoplethysmography, PPG, sensor (14) located in an ear-worn device (16);
determining signal quality of the received sensor data (12);
comparing the determined signal quality to a quality threshold;
if it is determined that the determined signal quality is greater than the quality threshold, providing the received sensor data (12) to a first processing pipeline (18) for determining, at least, pulse related biometric information (20); and
if it is determined that the determined signal quality is less than the quality threshold, providing the received sensor data (12) to a second, different processing pipeline (22) for determining, at least, user posture and/or movement related biometric information (26);
wherein the first processing pipeline performs at least one of: determining at least one vital sign of the user and user authentication, and wherein the second, different processing pipeline performs at least one of: facial expression recognition and activity recognition.

14. A computer program (836) as claimed in claim 13, wherein determining signal quality of the received sensor data comprises determining if morphological features (32) of the sensor data (12) are preserved.

## Patentansprüche

1. Einrichtung (10), umfassend Mittel zum:
Empfangen von Sensordaten (12) eines Benutzers von einem Photoplethysmographiesensor, PPG-Sensor (14), der sich in einem am Ohr getragenen Gerät (16) befindet;
Bestimmen einer Signalqualität der empfangenen Sensordaten (12);
Vergleichen der bestimmten Signalqualität mit einem Qualitätsschwellenwert;
falls bestimmt wird, dass die bestimmte Signalqualität größer als der Qualitätsschwellenwert ist, Bereitstellen der empfangenen Sensordaten (12) an eine erste Verarbeitungspipeline (18) zum Bestimmen von mindestens pulsbezogenen biometrischen Informationen (20); und
falls bestimmt wird, dass die bestimmte Signalqualität kleiner als der Qualitätsschwellenwert ist, Bereitstellen der empfangenen Sensordaten an eine zweite, verschiedene Verarbeitungspipeline (22) zum Bestimmen von mindestens benutzerkörperhaltungs- und/oder bewegungsbezogenen biometrischen Informationen (26);
wobei die erste Verarbeitungspipeline konfiguriert ist, um mindestens eines durchzuführen von: Bestimmen von mindestens einem Vitalzeichen des Benutzers und einer Benutzerauthentifizierung, und wobei die zweite, verschiedene Verarbeitungspipeline konfiguriert ist, um mindestens eines durchzuführen von: Gesichtsausdruckserkennung und Aktivitätserkennung.

2. Einrichtung nach Anspruch 1, wobei die Mittel konfiguriert sind, um ein Abtasten der empfangenen Sensordaten (12) in einem zeitlichen Fenster durchzuführen, um abgetastete Sensordaten (30) bereitzustellen, wobei das Bestimmen der Signalqualität an den abgetasteten Sensordaten durchgeführt wird.

3. Einrichtung nach einem der vorstehenden Ansprüche, wobei das Bestimmen der Signalqualität der empfangenen Sensordaten das Bestimmen umfasst, ob morphologische Merkmale (32) der Sensordaten (12) erhalten sind.

4. Einrichtung nach einem der vorstehenden Ansprüche, wobei das Bestimmen der Signalqualität das Bestimmen mindestens einer Signalmetrik für die empfangenen Sensordaten (12) umfasst.

5. Einrichtung nach einem der vorstehenden Ansprüche, wobei die mindestens eine Signalmetrik mindestens eines umfasst von: einer systolischen Phase, einer diastolischen Phase, einem Verhältnis zwischen systolischer und diastolischer Phase, einer Pulsbreite, einer Anstiegszeit, einem Perfusionsindex, einer dominanten Frequenz, einer spektralen Kurtosis, einer Spitze-zu-Spitze-Größenvarianz und einer Spitzenzeitintervallvarianz.

6. Einrichtung nach Anspruch 4 oder 5, wobei das Vergleichen der bestimmten Signalqualität mit einem Qualitätsschwellenwert das Vergleichen der bestimmten mindestens einen Signalmetrik mit mindestens einer Referenzsignalmetrik umfasst, die für einen Benutzer bestimmt ist, dessen Gesicht im Wesentlichen still und entspannt ist.

7. Einrichtung nach einem der vorstehenden Ansprüche, wobei die Mittel konfiguriert sind zum:
Empfangen erster Sensordaten (12) von einem ersten PPG-Sensor (14), der sich in einem ersten am Ohr getragenen Gerät (16) befindet, das an und/oder in einem ersten Ohr des Benutzers getragen wird;
Empfangen zweiter Sensordaten (12) von einem zweiten PPG-Sensor (14), der sich in einem zweiten am Ohr getragenen Gerät (16) befindet, das an und/oder in einem zweiten Ohr des Benutzers getragen wird; und
separates Bestimmen der Signalqualität der empfangenen ersten Sensordaten und der empfangenen zweiten Sensordaten, um separat zu bestimmen, an welche von der ersten und der zweiten Verarbeitungspipeline (18, 22) die ersten Sensordaten und die zweiten Sensordaten bereitgestellt werden sollen.

8. Elektronisches Gerät (34), umfassend eine Einrichtung (10) nach mindestens einem der Ansprüche 1 bis 7.

9. Verfahren (300), umfassend:
Empfangen (302) von Sensordaten (12) eines Benutzers von einem Photoplethysmographiesensor, PPG-Sensor (14), der sich in einem am Ohr getragenen Gerät (16) befindet;
Bestimmen (304) der Signalqualität der empfangenen Sensordaten (12);
Vergleichen (306) der bestimmten Signalqualität mit einem Qualitätsschwellenwert;
falls bestimmt wird, dass die bestimmte Signalqualität größer als der Qualitätsschwellenwert ist, Bereitstellen (308) der empfangenen Sensordaten (12) an eine erste Verarbeitungspipeline (18) zum Bestimmen von mindestens pulsbezogenen biometrischen Informationen (20); und
falls bestimmt wird, dass die bestimmte Signalqualität kleiner als der Qualitätsschwellenwert ist, Bereitstellen (310) der empfangenen Sensordaten (12) an eine zweite, verschiedene Verarbeitungspipeline (22) zum Bestimmen von mindestens benutzerkörperhaltungs- und/oder bewegungsbezogenen biometrischen Informationen (26);
wobei die erste Verarbeitungspipeline mindestens eines durchführt von: Bestimmen von mindestens einem Vitalzeichen des Benutzers und einer Benutzerauthentifizierung, und wobei die zweite, verschiedene Verarbeitungspipeline mindestens eines durchführt von: Gesichtsausdruckserkennung und Aktivitätserkennung.

10. Verfahren nach Anspruch 9, umfassend:
Abtasten der empfangenen Sensordaten (12) in einem zeitlichen Fenster, um abgetastete Sensordaten (30) bereitzustellen, wobei das Bestimmen der Signalqualität an den abgetasteten Sensordaten durchgeführt wird.

11. Verfahren nach Anspruch 9 oder 10, wobei das Bestimmen der Signalqualität der empfangenen Sensordaten das Bestimmen umfasst, ob morphologische Merkmale (32) der Sensordaten (12) erhalten sind.

12. Verfahren nach Anspruch 9, 10 oder 11, wobei das Bestimmen der Signalqualität das Bestimmen mindestens einer Signalmetrik für die empfangenen Sensordaten (12) umfasst.

13. Computerprogramm, umfassend Anweisungen zum Veranlassen einer Einrichtung (10) zum Durchführen von:
Empfangen von Sensordaten (12) eines Benutzers von einem Photoplethysmographiesensor, PPG-Sensor (14), der sich in einem am Ohr getragenen Gerät (16) befindet;
Bestimmen einer Signalqualität der empfangenen Sensordaten (12);
Vergleichen der bestimmten Signalqualität mit einem Qualitätsschwellenwert;
falls bestimmt wird, dass die bestimmte Signalqualität größer als der Qualitätsschwellenwert ist, Bereitstellen der empfangenen Sensordaten (12) an eine erste Verarbeitungspipeline (18) zum Bestimmen von mindestens pulsbezogenen biometrischen Informationen (20); und
falls bestimmt wird, dass die bestimmte Signalqualität kleiner als der Qualitätsschwellenwert ist, Bereitstellen der empfangenen Sensordaten (12) an eine zweite, verschiedene Verarbeitungspipeline (22) zum Bestimmen von mindestens benutzerkörperhaltungs- und/oder bewegungsbezogenen biometrischen Informationen (26);
wobei die erste Verarbeitungspipeline mindestens eines durchführt von: Bestimmen von mindestens einem Vitalzeichen des Benutzers und einer Benutzerauthentifizierung, und wobei die zweite, verschiedene Verarbeitungspipeline mindestens eines durchführt von: Gesichtsausdruckserkennung und Aktivitätserkennung.

14. Computerprogramm (836) nach Anspruch 13, wobei das Bestimmen der Signalqualität der empfangenen Sensordaten das Bestimmen umfasst, ob morphologische Merkmale (32) der Sensordaten (12) erhalten sind.

## Revendications

1. Appareil (10) comprenant des moyens destinés à :
recevoir des données de capteur (12) d'un utilisateur à partir d'un capteur de photopléthysmographie, PPG (14) situé dans un dispositif porté sur l'oreille (16) ;
déterminer une qualité de signal des données de capteur (12) reçues ;
comparer la qualité de signal déterminée à un seuil de qualité ;
s'il est déterminé que la qualité de signal déterminée est supérieure au seuil de qualité, fournir les données de capteur (12) reçues à un premier pipeline de traitement (18) pour déterminer, au moins, des informations biométriques relatives au pouls (20) ; et
s'il est déterminé que la qualité de signal déterminée est inférieure au seuil de qualité, fournir les données de capteur reçues à un second pipeline de traitement (22) différent pour déterminer, au moins, des informations biométriques relatives à la posture et/ou au mouvement de l'utilisateur (26) ;
dans lequel le premier pipeline de traitement est configuré pour effectuer au moins l'un parmi : la détermination d'au moins un signe vital de l'utilisateur et d'une authentification d'utilisateur, et dans lequel le second pipeline de traitement différent est configuré pour effectuer au moins l'une parmi : une reconnaissance d'expression faciale et une reconnaissance d'activité.

2. Appareil selon la revendication 1, dans lequel les moyens sont configurés pour effectuer un échantillonnage des données de capteur (12) reçues dans une fenêtre temporelle afin de fournir des données de capteur échantillonnées (30), dans lequel la détermination de la qualité de signal est effectuée sur les données de capteur échantillonnées.

3. Appareil selon l'une quelconque revendication précédente, dans lequel la détermination de la qualité de signal des données de capteur reçues comprend le fait de déterminer si des caractéristiques morphologiques (32) des données de capteur (12) sont préservées.

4. Appareil selon l'une quelconque revendication précédente, dans lequel la détermination de la qualité de signal comprend la détermination d'au moins une mesure de signal pour les données de capteur (12) reçues.

5. Appareil selon l'une quelconque revendication précédente, dans lequel l'au moins une mesure de signal comprend au moins l'un parmi : une phase systolique, une phase diastolique, un rapport entre les phases systolique et diastolique, une largeur d'impulsion, un temps de montée, un indice de perfusion, une fréquence dominante, un aplatissement spectral, une variance de magnitude crête à crête et une variance d'intervalle de temps de crête.

6. Appareil selon la revendication 4 ou 5, dans lequel la comparaison de la qualité de signal déterminée à un seuil de qualité comprend la comparaison de l'au moins une mesure de signal déterminée à au moins une mesure de signal de référence déterminée pour un utilisateur dont le visage est sensiblement immobile et détendu.

7. Appareil selon l'une quelconque revendication précédente, dans lequel les moyens sont configurés pour :
recevoir de premières données de capteur (12) à partir d'un premier capteur PPG (14) situé dans un premier dispositif porté sur l'oreille (16) porté sur et/ou dans une première oreille de l'utilisateur ;
recevoir des secondes données de capteur (12) à partir d'un second capteur PPG (14) situé dans un second dispositif porté sur l'oreille (16) porté sur et/ou dans une seconde oreille de l'utilisateur ; et
déterminer séparément une qualité de signal des premières données de capteur reçues et des secondes données de capteur reçues pour déterminer séparément auquel des premier et second pipelines de traitement (18, 22) les premières données de capteur et les secondes données de capteur doivent être fournies.

8. Dispositif électronique (34) comprenant un appareil (10) selon au moins l'une des revendications 1 à 7.

9. Procédé (300) comprenant :
la réception (302) de données de capteur (12) d'un utilisateur à partir d'un capteur de photopléthysmographie, PPG (14) situé dans un dispositif porté sur l'oreille (16) ;
la détermination (304) d'une qualité de signal des données de capteur (12) reçues ;
la comparaison (306) de la qualité de signal déterminée à un seuil de qualité ;
s'il est déterminé que la qualité de signal déterminée est supérieure au seuil de qualité, la fourniture (308) des données de capteur (12) reçues à un premier pipeline de traitement (18) pour déterminer, au moins, des informations biométriques relatives au pouls (20) ; et
s'il est déterminé que la qualité de signal déterminée est inférieure au seuil de qualité, la fourniture (310) des données de capteur (12) reçues à un second pipeline de traitement (22) différent pour déterminer, au moins, des informations biométriques relatives à la posture et/ou au mouvement de l'utilisateur (26) ;
dans lequel le premier pipeline de traitement effectue au moins l'un parmi : la détermination d'au moins un signe vital de l'utilisateur et d'une authentification d'utilisateur, et dans lequel le second pipeline de traitement différent effectue au moins l'un parmi : une reconnaissance d'expression faciale et une reconnaissance d'activité.

10. Procédé selon la revendication 9, comprenant :
l'échantillonnage des données de capteur (12) reçues dans une fenêtre temporelle pour fournir des données de capteur échantillonnées (30), dans lequel la détermination de la qualité de signal est effectuée sur les données de capteur échantillonnées.

11. Procédé selon la revendication 9 ou 10, dans lequel la détermination d'une qualité de signal des données de capteur reçues comprend le fait de déterminer si des caractéristiques morphologiques (32) des données de capteur (12) sont préservées.

12. Procédé selon la revendication 9, 10 ou 11, dans lequel la détermination de la qualité de signal comprend la détermination d'au moins une mesure de signal pour les données de capteur (12) reçues.

13. Programme informatique comprenant des instructions permettant d'amener un appareil (10) à effectuer :
la réception de données de capteur (12) d'un utilisateur à partir d'un capteur de photopléthysmographie, PPG (14) situé dans un dispositif porté sur l'oreille (16) ;
la détermination d'une qualité de signal des données de capteur (12) reçues ;
la comparaison de la qualité de signal déterminée à un seuil de qualité ;
s'il est déterminé que la qualité de signal déterminée est supérieure au seuil de qualité, la fourniture des données de capteur (12) reçues à un premier pipeline de traitement (18) pour déterminer, au moins, des informations biométriques relatives au pouls (20) ; et
s'il est déterminé que la qualité de signal déterminée est inférieure au seuil de qualité, la fourniture des données de capteur (12) reçues à un second pipeline de traitement (22) différent pour déterminer, au moins, des informations biométriques relatives à la posture et/ou au mouvement de l'utilisateur (26) ;
dans lequel le premier pipeline de traitement effectue au moins l'un parmi : la détermination d'au moins un signe vital de l'utilisateur et d'une authentification d'utilisateur, et dans lequel le second pipeline de traitement différent effectue au moins l'un parmi : une reconnaissance d'expression faciale et une reconnaissance d'activité.

14. Programme informatique (836) selon la revendication 13, dans lequel la détermination d'une qualité de signal des données de capteur reçues comprend le fait de déterminer si des caractéristiques morphologiques (32) des données de capteur (12) sont préservées.
